# FASCICULE DE BREVET EUROPEEN

(11) **EP 0 921 758 B1**
(45) Date de publication et mention de la délivrance du brevet: **06.10.2004**
(21) Numéro de dépôt: 97934577.4
(22) Date de dépôt: 16.07.1997
(51) Int. Cl.: A61B 10/00

(54) **DISPOSITIF DE PINCEMENT, NOTAMMENT DU TYPE PINCE A BIOPSIE**
PINZETTE, INSBESONDERE ZUR BIOPSIE
CLAMPING DEVICE, PARTICULARLY A BIOPSY FORCEPS

(30) Priorité: 18.07.1996 FR 9609023
(43) Date de publication de la demande: 16.06.1999
(73) Titulaire: EUROBIOPSY, 69430 Régnié Durette (FR)
(72) Inventeur: Sabin, Pierre, 76230 Bois-Guillaume (FR); Hugueny, Jean-Marie, 69430 Régnié-Durette (FR); Sabin, Jean-Louis, 97220 Sainte Luce (FR)
(74) Mandataire: Schmitt, John
(86) Numéro de dépôt international: PCT/FR1997/001324
(87) Numéro de publication internationale: WO 1998/003116

(56) Documents cités:
- EP-A- 0 380 874
- WO-A-95/07662
- US-A- 3 895 636
- US-A- 5 275 615
- US-A- 5 290 309

## Description

La présente invention a trait à un dispositif de pincement, tel que, notamment, une pince à biopsie, c'est-à-dire une pince ayant deux mors susceptibles de s'écarter, de se rapprocher et, lors du rapprochement, de couper ou détacher un échantillon corporel qui se trouve pris entre les deux mors de la pince et qui peut ensuite être récupéré à l'extérieur de l'organisme. Un tel dispositif est connu de EP-A-0 380 874.

L'invention s'étend cependant également à d'autres appareils utilisables en médecine ou en chirurgie, tel que, par exemple, les appareils de pinçage sans effet dé coupe, par exemple pour assurer un effet de clampage, ou des écarteurs, ou encore des ciseaux à usage chirurgical, ou tout autre appareil de ce genre possédant deux éléments, dont l'un, au moins, est capable de s'écarter et de se rapprocher de l'autre.

De façon typique, les pinces à biopsie actuellement connues comportent deux mors susceptibles de s'écarter ou de se rapprocher, articulés à l'extrémité d'un tube ou d'un gaine rigide ou souple allongé, à l'intérieur de laquelle peut coulisser un câble muni, à son autre extrémité, de moyens de manoeuvre par l'opérateur. Ces dispositifs comportent des biellettes articulées destinées à assurer une amplification de mouvement entre le moyen de commande constitué par le câble et les mors proprement dit.

Ces pinces sont mécaniquement complexes et comportent au moins une dizaine de pièces. Elles sont donc coûteuses, difficiles à monter et sujettes à l'usure et aux pannes. Elles entraînent, au niveau de la liaison avec le câble, des effets de torsion induisant une fatigue du câble pouvant provoquer sa rupture. D'autres pièces, également, peuvent être soumises à des contraintes exagérées, de sorte que finalement, ces pinces présentent un grand nombre d'inconvénients.

La présente invention se propose de remédier à ces inconvénients et de fournir un appareil de pincement, notamment une pince à biopsie, de conception extrêmement simple, avec un nombre très limité de pièces, supprimant tout effort excessif susceptible de conduire à une usure des pièces ou à une torsion du câble de manoeuvre, et permettant d'obtenir, d'une façon simple, des mouvements complexes, si on le désire, tout en gardant une très grande précision des mouvements des mors.

L'invention a pour objet un appareil de pincement, notamment du type pince à biopsie, comprenant, à l'extrémité d'une gaine rigide ou non, dans laquelle peut coulisser un élément allongé de manoeuvre, de préférence un câble, deux mors, dont l'un, au moins, est susceptible de s'écarter et de se rapprocher de l'autre, lorsque ledit élément de manoeuvre coulisse axialement dans l'extrémité de la gaine, caractérisé en ce que le ou lesdits mors susceptibles de s'écarter et de se rapprocher présentent, dans le prolongement d'une partie de mors faisant office de mâchoires proprement dites, un bras se rapprochant progressivement de l'axe de la gaine de façon à former une première surface inclinée, interne, se rapprochant progressivement de l'axe et faisant face à celui-ci, une deuxième surface inclinée, externe, se rapprochant également progressivement de l'axe et faisant face à la surface interne de l'extrémité de la gaine, ledit bras présentant un renflement convexe susceptible de glisser sensiblement contre ladite surface interne de l'extrémité de la gaine, ledit renflement présentant, au regard de l'axe, une cavité creuse concave sensiblement sphérique, en ce que ladite gaine présente, vers son extrémité au-delà de laquelle s'étend ladite mâchoire proprement dite, un élément transversal, tel qu'une pige contre laquelle vient glisser ladite surface interne dudit bras, et en ce que ledit élément allongé de manoeuvre se termine par une rotule logée dans ladite cavité sphérique, de sorte que lorsque ledit élément allongé de manoeuvre ou câble est déplacé vers l'extrémité libre de la gaine, il repousse ledit mors au-delà de l'extrémité, amenant ainsi la surface interne dudit bras, qui glisse sur ledit élément transversal, à pivoter par effet de rampe, en écartant la mâchoire du mors de l'autre mâchoire, ledit écartement étant autorisé par l'inclinaison de ladite surface externe qui sort de l'extrémité de la surface interne de la gaine, ledit renflement du bras pivotant, dans ce mouvement, autour de ladite rotule tout en restant guidé dans ladite gaine, le mouvement inverse de l'élément de manoeuvre provoquant le rapprochement du mors par un mouvement inverse.

De préférence l'extrémité de la gaine, notamment lorsque la gaine est souple, est formée d'un élément rigide fixé sur une extrémité de la gaine proprement dite par une extrémité et dont l'autre extrémité, libre, de l'élément rigide porte l'élément transversal ou pige, de préférence au niveau de deux prolongements en forme de créneaux de façon que l'axe géométrique de la pige soit situé au voisinage du niveau des fonds des créneaux.

De préférence ladite surface interne de l'extrémité de la gaine, ou de son élément d'extrémité rigide, est plane et les surfaces interne et externe du ou des bras étant alors des surfaces planes ou géométriquement cylindriques avec une génératrice parallèle à ladite surface interne de l'extrémité de la gaine et à la pige, la surface externe du renflement du bras étant également cylindrique.

Avantageusement, la section de l'extrémité de la gaine, ou de son élément d'extrémité rigide délimite intérieurement un rectangle, notamment un carré, ladite surface interne de l'extrémité de la gaine, ou de son élément d'extrémité rigide étant engendrée par un côté du rectangle.

On peut, ainsi grâce à l'invention, donner à la surface interne du bras de mors, un profil rectiligne, ou au contraire variable, se rapprochant progressivement de l'axe de la gaine au fur et à mesure que l'on s'enfonce dans la gaine, ce qui permet de donner au mouvement d'écartement du mors une cinématique correspondant à la forme de la rampe ainsi réalisée. La face externe dudit bras est alors conformée, en se rapprochant également de l'axe, de préférence de façon à constamment glisser sur l'extrémité de la surface interne de la gaine et éviter ainsi un débattement libre du bras entre ladite pige et la surface interne de la gaine.

Le mors présente, au-delà du bras, la partie formant la mâchoire proprement dite, qui peut être de toute forme appropriée à l'usage que l'on veut en faire.

La mâchoire de mors peut, par exemple, être réalisée sous forme d'une cuiller à pourtour coupant dans le cas d'une pince de biopsie.

En variante la forme de la mâchoire peut être celle d'un mors plat, par exemple pour réaliser un effet de serrage ou de clampage.

En variante encore la mâchoire peut se présenter sous forme d'une lame coupante de ciseau pour réaliser un instrument de sectionnement.

En général les deux mors sont symétriques et animés de mouvements parfaitement symétriques par rapport à un plan passant par l'axe de l'extrémité de la gaine et l'axe de la pige.

Cependant, dans une variante, l'un des mors peut être réalisé de façon à rester fixe en rotation et n'être animé que d'un mouvement de coulissement pendant que l'autre est animé d'un mouvement de coulissement et de pivotement par rapport à la gaine.

Lors de l'utilisation, on peut souhaiter que le mors, en s'écartant, se déplace également axialement par rapport à l'élément à traiter, par exemple un organe. Dans ce cas la gaine restera fixe par rapport à l'organe à traiter et le moyen de manoeuvre tel que le câble sera animé d'un mouvement de translation dans la gaine.

Au contraire on peut souhaiter que les mors soient animés uniquement d'un mouvement d'écartement et de rapprochement par rapport à l'objet ou organe à traiter et dans ce cas c'est l'élément de manoeuvre qui restera fixe tandis que la gaine sera déplacée en translation le long de cet élément de manoeuvre allongé.

A cet effet, l'élément de manoeuvre et la gaine comportent des moyens adaptés pour respectivement jouer tour à tour le rôle de moyens de maintien en position de l'un de l'élément de manoeuvre ou de la gaine par rapport à l'organe à traiter et le rôle de moyens de déplacement de l'autre.

On comprend que l'on a ainsi réalisé un dispositif de pincement d'une très grande simplicité mécanique et qui comprend un nombre très limité de pièces, à savoir les deux mors, la pige transversale, la rotule qui termine la gaine, et éventuellement un tronçon tubulaire rapporté sur l'extrémité de la gaine et formant la surface interne de guidage et recevant, à son extrémité, la pige.

Il en résulte un montage et un démontage facile, une simplicité d'entretien et une quasi suppression des risques de rupture ou de panne, d'autant plus que l'élément de manoeuvre tel qu'un câble n'est soumis à aucune contrainte de torsion, sa position angulaire autour de l'axe de la gaine étant entièrement libre, par glissement de la rotule à l'intérieur de la cavité concave des bras de mors.

D'autres avantages et caractéristiques de l'invention apparaîtront à la lecture de la description suivante, faite à titre d'exemple non limitatif et se référant au dessin annexé dans lequel :

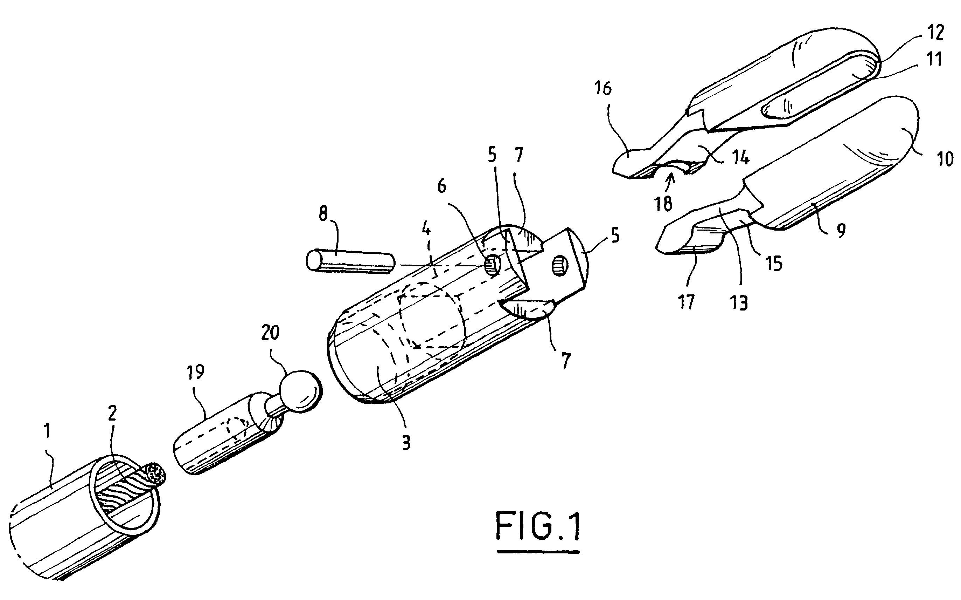

- la figure 1 représente une vue en perspective éclatée du dispositif selon l'invention,
- la figure 2 représente une vue en coupe axiale, dans un plan perpendiculaire à la pige, du dispositif,
- la figure 3 représente une vue en coupe axiale du dispositif dans un plan axial de la pige, et
- la figure 4 représente une vue analogue à la figure 2 en position écartée des mors.

La pince à biopsie représentée sur les figures comporte une gaine allongée 1 à l'intérieur de laquelle peut se déplacer longitudinalement un câble semi rigide 2 dont l'une des extrémités, non représentée, est reliée à une poignée de manoeuvre de type usuel dans ce genre de pince. Avantageusement, il est prévu des moyens de maintien en position par rapport à l'organe à traiter de l'un ou l'autre de la gaine et du câble et des moyens de déplacement de celui non maintenu de la gaine et du câble.

A son extrémité la gaine 1 présente un élément tubulaire métallique 3, dont la surface extérieure est cylindrique, et qui comporte un passage central de section rectangulaire ou carrée 4. A son extrémité éloignée de la gaine, la pièce cylindrique 3 est découpée en forme de créneaux de façon à former deux prolongements 5 traversés par des trous alignés 6 dont l'axe géométrique commun est situé légèrement au dessus du plan des deux bords plus bas 7 des créneaux. Ainsi, la partie inférieure des trous 6 se situe sensiblement dans le plan de ces bords les plus bas. Dans les trous 6 est chassée une pige cylindrique 8 qui s'étend ainsi transversalement à l'extrémité supérieure du passage de section carrée 4.

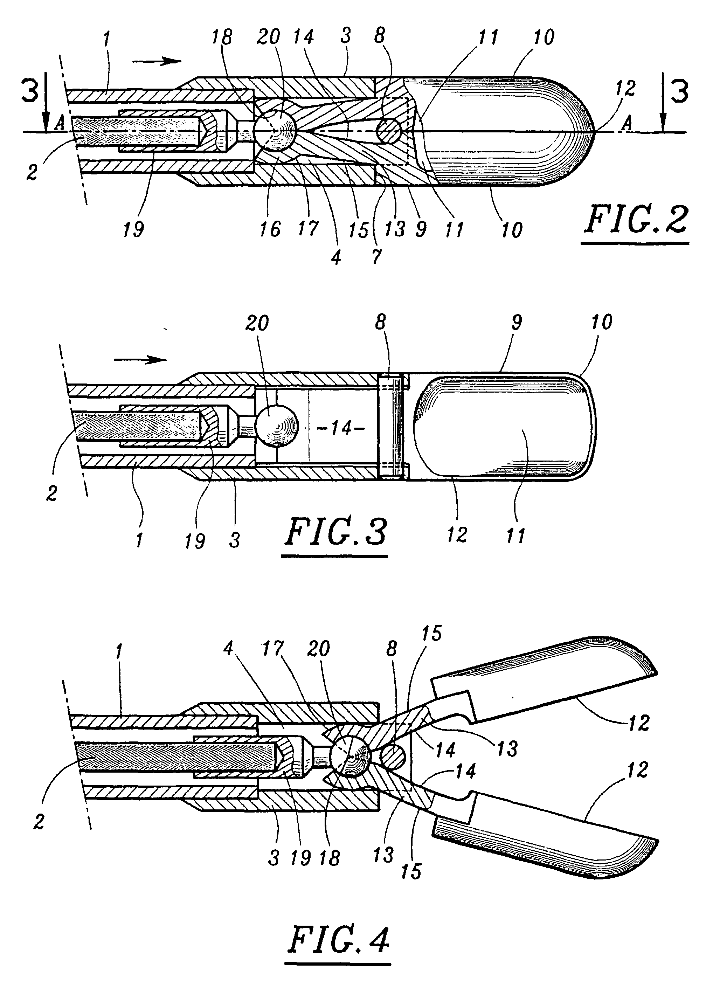

La pince comporte deux mors 9 identiques dont les parties formant les mâchoires proprement dites 10, s'étendent au-delà de l'extrémité crénelée de la pièce tubulaire 3 et présentent, d'une façon usuelle, des cavités 11 bordées par une lèvre coupante périphérique 12. Les mors 9 se prolongent chacun, à l'intérieur de la pièce 3, par un bras 13 qui passe entre la pige 8 et la surface interne plane du passage 4 au regard de la pige. Le bras 13 présente une surface interne 14 engendrée par une génératrice perpendiculaire au plan de la figure 2 et qui, depuis la tige 8, se rapproche progressivement de l'axe A-A du dispositif jusqu'à venir pratiquement tangenter cet axe. Au-dessus de la pige, dans la position représentée sur la figure 2, la surface 14 s'incurve à nouveau en direction de l'axe et cette fois-ci vers le haut de façon à sensiblement entourer la pige comme on le voit bien sur la figure 2.

Les bras 13 présentent également une surface extérieure 15, qui tend également à se rapprocher de l'axe A-A lorsque l'on descend vers le bas et qui est telle que l'épaisseur du bras 13, dans la direction perpendiculaire à l'axe A-A, comprise dans le plan de la figure 2, tend progressivement à diminuer, cette épaisseur étant presque égale à la distance située entre la pige 8 et la paroi interne plane de la pièce 3 au niveau du bord 7. Le bras 13 présente un renflement convexe d'extrémité 16 dont la surface externe 17 a sensiblement la forme d'un cylindre à base circulaire, dont le diamètre est un peu inférieur à la distance séparant les deux surfaces internes planes, en regard, de la pièce 3. Le renflement 16 présente, à l'intérieur, une cavité 18 de forme sphérique.

L'extrémité du câble 2 porte un embout 19 serti sur le câble et présentant, à son extrémité libre, une tête sphérique ou rotule 20 qui est reçue dans la réunion des deux cavités sphériques 18 des deux mors 9.

Pour le montage, les deux mors 9 sont placés convenablement sur la rotule 20 puis ils sont introduits dans la pièce 3 et on met enfin en place la pige 8.

Lorsque, partant de la position de pince fermée, représentée sur les figures 2 et 3, on repousse lé câble vers le haut, dans le sens de la flèche, la rotule 20 repousse les deux mors vers le haut, de sorte que, rapidement, les surfaces 14, qui viennent glisser sur la pige 8, produisent un effet de rampe qui tend à écarter les deux bras 13 en les faisant pivoter autour de la rotule 20, tout en maintenant les deux renflements 16 assemblées autour de la rotule du fait que ces extrémités restent guidées dans le passage carré 4.

Ce mouvement de basculement des bras est progressivement autorisé du fait de l'inclinaison de la surface externe 15 des bras qui évite un coincement et guide les bras au fur et à mesure que ceux-ci montent au-dessus des rebords 7. En fin de parcours le mouvement est arrêté par la présence de la pige et les deux mors se trouvent en position d'ouverture maximale.

La fermeture des mors s'effectue en manoeuvrant le câble 2 dans le sens opposé à celui de la flèche.

On comprend qu'on a ainsi réalisé une pince à biopsie composée d'un très petit nombre d'éléments et dont la simplicité mécanique permet de supprimer pratiquement tout risque de panne. En outre les efforts mécaniques sur une telle pince, qui peut être très miniaturisée, sont parfaitement répartis. Enfin une rotation du câble 2 n'entraîne aucun effet de coincement puisque la rotule 20 peut tourner librement dans son siège sphérique formée par la réunion des deux cavités 18.

De plus, la tête de pince à biopsie décrite ici présente une faible longueur axiale grâce à la simplicité de sa construction. Ainsi, elle peut circuler dans des tubes de guidage présentant de faibles rayons de courbure sans risque de coincement.

En variante, la tête de pince peut comporter des mors asymétriques, dont l'un porte par exemple une dent unique et l'autre porte deux dents délimitant un espace de réception pour la dent unique du mors complémentaire lorsque la pince est fermée.

## Revendications

1. Appareil de pincement, notamment du type pince à biopsie, comprenant, à l'extrémité d'une gaine (1,3) rigide ou non, dans laquelle peut coulisser un élément allongé de manoeuvre (2), de préférence un câble, deux mors (9), dont l'un, au moins, est susceptible de s'écarter et de se rapprocher de l'autre, lorsque ledit élément de manoeuvre (2) coulisse axialement dans l'extrémité de la gaine (1, 3), le ou lesdits mors (9) susceptibles de s'écarter et de se rapprocher présentant, dans le prolongement d'une partie de mors faisant office de mâchoires proprement dites (10), un bras (13) se rapprochant progressivement de l'axe de la gaine (1, 3) de façon à former une première surface inclinée (14), interne, se rapprochant progressivement de l'axe (A-A) et faisant face à celui-ci, une deuxième surface inclinée (15), externe, se rapprochant également progressivement de l'axe (A-A) et faisant face à la surface interne de l'extrémité de la gaine (1, 3), ledit bras (13) présentant un renflement convexe (16) susceptible de glisser sensiblement contre ladite surface interne de l'extrémité de la gaine (1,3), ladite gaine (1, 3) présentant, vers son extrémité au-delà de laquelle s'étend ladite mâchoire proprement dite (10), un élément transversal (8), tel qu'une pige contre laquelle vient glisser ladite surface interne (14) dudit bras (13), de sorte que lorsque ledit élément allongé de manoeuvre (2) ou câble est déplacé vers l'extrémité libre de la gaine (1, 3), il repousse ledit mors (9) au-delà de l'extrémité, amenant ainsi la surface interne (14) dudit bras (13), qui glisse sur ledit élément transversal (8), à pivoter par effet de rampe, en écartant la mâchoire du mors de l'autre mâchoire, ledit écartement étant autorisé par l'inclinaison de ladite surface externe (15) qui sort de l'extrémité de la surface interne de la gaine (1, 3), **caractérisé en ce que** ledit renflement (16) présente, au regard de l'axe (A-A), une cavité creuse concave (18) sensiblement sphérique, et **en ce que** ledit élément allongé de manoeuvre (2) se termine par une rotule (20) logée dans ladite cavité sphérique (18), ledit renflement (16) du bras pivotant, dans ce mouvement, autour de ladite rotule (20) tout en restant guidé dans ladite gaine (1, 3), le mouvement inverse de l'élément de manoeuvre (2) provoquant le rapprochement du mors (9) par un mouvement inverse.

2. Appareil selon la revendication 1, **caractérisé en ce que** la gaine (1,3), notamment lorsque la gaine est souple, est formée d'un élément rigide (3) fixé sur une extrémité de la gaine (1) proprement dite par une extrémité et dont l'autre extrémité, libre, de l'élément rigide (3) porte l'élément transversal (8) ou pige.

3. Appareil selon la revendication 2, **caractérisé en ce que** l'élément transversal (8) ou pige est porté au niveau de deux prolongements (5) en forme de créneaux de façon que l'axe géométrique de la pige (8) soit situé au voisinage du niveau des fonds (7) des créneaux.

4. Appareil selon l'une quelconque des revendications précédentes, **caractérisé en ce que** ladite surface interne de l'extrémité de la gaine (1), ou de son élément d'extrémité rigide (3), est plane et les surfaces interne (14) et externe (15) du ou des bras (13) étant alors des surfaces planes ou géométriquement cylindriques avec une génératrice parallèle à ladite surface interne de l'extrémité de la gaine et à la pige (8), la surface (17) externe du renflement (16) du bras étant également cylindrique.

5. Appareil selon la revendication 4, **caractérisé en ce que** la section de l'extrémité de la gaine (1), ou de son élément d'extrémité rigide (3) délimite intérieurement un rectangle, notamment un carré, ladite surface interne de l'extrémité de la gaine (1), ou de son élément d'extrémité rigide (3) étant engendrée par un côté du rectangle.

6. Appareil selon l'une quelconque des revendications précédentes, **caractérisé en ce que** lesdites mâchoires proprement dites comportent des cavités (11) bordées par une lèvre coupante périphérique (12).

7. Appareil selon l'une quelconque des revendications précédentes, **caractérisé en ce qu'**il comporte deux mors (9) identiques.

8. Appareil selon l'une quelconque des revendications 1 à 6, **caractérisé en ce qu'**il comporte deux mors asymétriques.

9. Appareil selon l'une quelconque des revendications précédentes, **caractérisé en ce que** l'élément de manoeuvre (2) et la gaine (3) comportent des moyens adaptés pour respectivement jouer tour à tour le rôle de moyens de maintien en position de l'un de l'élément de manoeuvre (2) ou de la gaine (3) par rapport à l'organe à traiter et le rôle de moyens de déplacement de l'autre.

## Patentansprüche

1. Klemmvorrichtung, insbesondere der Biopsiezangenart, mit am Ende einer gegebenenfalls starren Hülle (1, 3), in der ein längliches Betätigungselement (2), vorzugsweise ein Kabel, gleiten kann, zwei Spannbacken (9), von denen mindestens eine sich von der anderen entfernen und sich ihr nähern kann, wenn das Betätigungselement (2) axial in dem Ende der Hülle (1, 3) gleitet, wobei die Spannbacke(n) (9) die sich entfernen und annähern kann (können), in der Verlängerung eines Spannbackenteils, der als die eigentlichen Klemmbacken (10) fungiert, einen Arm (13) aufweist (aufweisen), der sich der Achse der Hülle (1, 3) allmählich nähert, um eine erste geneigte Innenfläche (14) zu bilden, die sich der Achse (A-A) allmählich nähert und zu ihr weist, wobei eine zweite geneigte Außenfläche (15) sich ebenfalls der Achse (A-A) allmählich nähert und zu der Innenfläche des Endes der Hülle (1, 3) weist, wobei der Arm (13) eine konvexe Verdickung (16) aufweist, die im Wesentlichen an der Innenfläche des Endes der Hülle (1, 3) gleiten kann, wobei die Hülle (1, 3) gegen ihr Ende, hinter dem sich die eigentliche Klemmbacke (10) erstreckt, ein Querelement (8), wie zum Beispiel einen Stab, aufweist, an dem die Innenfläche (14) des Arms (13) gleitet, so dass das längliche Betätigungselement (2) oder Kabel bei seiner Verschiebung zu dem freien Ende der Hülle (1, 3) hin die Spannbacke (9) über das Ende hinaus schiebt und so die Innenfläche (14) des Arms (13), die auf dem Querelement (8) gleitet, dazu veranlasst, durch Rampenwirkung zu schwenken, wobei die Klemmbacke der Spannbacke von der anderen Klemmbacke entfernt wird, wobei das Entfernen durch die Neigung der Außenfläche (15), die aus dem Ende der Innenfläche der Hülle (1, 3) heraustritt, gestattet wird, **dadurch gekennzeichnet, dass** die Verdickung (16) gegenüber der Achse (A-A) einen konkaven Hohlraum (18) aufweist, der im Wesentlichen kugelförmig ist, und dass das längliche Betätigungselement (2) mit einem Kugelkopf (20) abschließt, der in dem kugelförmigen Hohlraum (18) untergebracht ist, wobei die Verdickung (16) des Arms bei dieser Bewegung um den Kugelkopf (20) schwenkt und dabei weiterhin in der Hülle (1, 3) geführt wird, wobei die umgekehrte Bewegung des Betätigungselements (2) das Annähern der Spannbacke (9) durch eine umgekehrte Bewegung bewirkt.

2. Vorrichtung nach Anspruch 1, **dadurch gekennzeichnet, dass** die Hülle (1, 3), insbesondere, wenn sie flexibel ist, durch ein starres Element (3) gebildet wird, das, eigentlich durch ein Ende, an einem Ende der Hülle (1) befestigt ist, wobei das andere, freie Ende des starren Elements (3) das Querelement (8) oder den Stab trägt.

3. Vorrichtung nach Anspruch 2, **dadurch gekennzeichnet, dass** das Querelement (8) oder der Stab an zwei zinnenförmigen Verlängerungen (5) getragen wird, so dass sich die geometrische Achse des Stabs (8) in der Nähe der Böden (7) der Zinnen befindet.

4. Vorrichtung nach einem der vorhergehenden Ansprüche, **dadurch gekennzeichnet, dass** die Innenfläche des Endes der Hülle (1) oder seines starren Endelements (3) eben ist und die Innenfläche (14) und die Außenfläche (15) des Arms oder der Arme (13) dann ebene oder geometrisch zylindrische Flächen mit einer parallel zur Innenfläche des Endes der Hülle und zum Stab (8) verlaufenden Erzeugenden sind, wobei die Außenfläche (17) der Verdickung (16) des Arms ebenfalls zylindrisch ist.

5. Vorrichtung nach Anspruch 4, **dadurch gekennzeichnet, dass** der Querschnitt des Endes der Hülle (1) oder seines starren Endelements (3) innen ein Rechteck, insbesondere ein Quadrat, begrenzt, wobei die Innenfläche des Endes der Hülle (1) oder seines starren Endelements (3) durch eine Seite des Rechtecks erzeugt wird.

6. Vorrichtung nach einem der vorhergehenden Ansprüche, **dadurch gekennzeichnet, dass** die eigentlichen Klemmbacken Hohlräume (11) aufweisen, die von einer umlaufenden Schneidlippe (12) eingefasst sind.

7. Vorrichtung nach einem der vorhergehenden Ansprüche, **dadurch gekennzeichnet, dass** sie zwei identische Spannbacken (9) aufweist.

8. Vorrichtung nach einem der Ansprüche 1 bis 6, **dadurch gekennzeichnet, dass** sie zwei asymmetrische Spannbacken aufweist.

9. Vorrichtung nach einem der vorhergehenden Ansprüche, **dadurch gekennzeichnet, dass** das Betätigungselement (2) und die Hülle (3) Mittel aufweisen, die dazu ausgeführt sind, jeweils nacheinander die Rolle von Mitteln zum Festhalten des Betätigungselements (2) oder der Hülle (3) bezüglich des zu behandelnden Organs und die Rolle von Mitteln zur Verschiebung des (der) anderen davon zu spielen.

## Claims

1. Forceps instrument, in particular of the biopsy-forceps type, comprising, on the end of a rigid or flexible sheath (1,3), in which may slide an elongate manoeuvring element (2), preferably a cable, two jaw-pieces (9), one of which, at least, is capable of being moved away from and of being moved towards the other when the said manoeuvring element (2) slides axially in the end of the sheath (1,3), the said jaw-piece or jaw-pieces (9), capable of being moved away from and of being moved towards each other having, in the extension of a part of the jaw-pieces acting as the jaws proper (10), an arm (13), which goes progressively towards the axis of the sheath (1,3) so as to form a first, internal, inclined surface (14), going progressively towards the axis (A-A) and facing the latter, a second, external, inclined surface (15) also going progressively towards the axis (A-A) and facing the internal surface of the end of the sheath (1,3), the said arm (13) having a convex excrescence (16) capable of sliding substantially against the said internal surface of the end of the sheath (1,3), the said sheath (1,3) having, near its end beyond which the said jaw proper (10) extends, a transverse element (8), such as a rod against which the said internal surface (14) of the said arm (13) slides, so that, when the said elongate manoeuvring element (2) or cable is moved towards the free end of the sheath (1,3), it pushes the said jaw-piece (9) back beyond the end, thus causing the internal surface (14) of the said arm (13), which slides over the said transverse element (8), to pivot by a ramp effect, moving the jaw of the jaw-piece away from the other jaw, the said movement away from the other jaw being allowed by the inclination of the said external surface (15) which protrudes from the end of the internal surface of the sheath (1,3), **characterized in that** the said excrescence (16) has, facing the axis (A-A), a substantially spherical concave hollow cavity (18), and **in that** the said elongate manoeuvring element (2) terminates in a ball (20) housed in the said spherical cavity (18), the said excrescence (16) of the arm pivoting, during this movement, about the said ball (20) while still being guided in the said sheath (1,3), the reverse movement of the manoeuvring element (2) causing the jaw-piece (9) to come together by a reverse movement.

2. Instrument according to Claim 1, **characterized in that** the sheath (1,3), in particular when the sheath is flexible, is formed by a rigid element (3), fixed to one end of the sheath (1) proper by one end of the rigid element (3), the other, free end of which supports the transverse element (8) or rod.

3. Instrument according to Claim 2, **characterized in that** the transverse element (8) or rod is supported by two crenel-shaped extensions (5) so that the geometrical axis of the rod (8) is located near the bottom (7) of the crenels.

4. Instrument according to any one of the preceding claims, **characterized in that** the said internal surface of the end of the sheath (1), or of its rigid end element (3), is plane, the internal (14) and external (15) surfaces of the arm or arms (13) then being plane surfaces or geometrically cylindrical surfaces with a generatrix parallel to the said internal surface of the end of the sheath and to the rod (8), and the external surface (17) of the excrescence (16) of the arm also being cylindrical.

5. Instrument according to Claim 4, **characterized in that** the cross-section of the end of the sheath (1), or of its rigid end element (3), delimits internally a rectangle, in particular a square, the said internal surface of the end of the sheath (1), or of its rigid end element (3), being generated by one side of the rectangle.

6. Instrument according to any one of the preceding claims, **characterized in that** the said jaws proper have cavities (11) whose edges form a peripheral cutting lip (12).

7. Instrument according to any one of the preceding claims, **characterized in that** it has two identical jaw-pieces (9).

8. Instrument according to any one of Claims 1 to 6, **characterized in that** it has two asymmetric jaw-pieces.

9. Instrument according to any one of the preceding claims, **characterized in that** the manoeuvring element (2) and the sheath (3) include means which are designed to act, in turn, as means of holding one of the manoeuvring element (2) or the sheath (3) in position with respect to the organ to be treated and as means of moving the other of these, respectively.
